# EUROPEAN PATENT APPLICATION

(11) **EP 3 881 834 A1**
(43) Date of publication of application: **22.09.2021**
(21) Application number: 19871976.7
(22) Date of filing: 04.03.2019
(51) Int. Cl.: A61K 9/48, A61K 47/36

(54) **PULLULAN EMPTY HARD CAPSULE AND PREPARATION METHOD THEREFOR**

(30) Priority: 12.10.2018 CN 201811189150
(71) Applicant: Jiangsu Lefan Capsule Co., Ltd, Zhenjiang, Jiangsu 212100 (CN)
(72) Inventor: ZHANG, Yichi, Zhenjiang, Jiangsu 212100 (CN); PAN, Jie, Zhenjiang, Jiangsu 212100 (CN); FANG, Zhongming, Zhenjiang, Jiangsu 212100 (CN); LIU, Peiyong, Zhenjiang, Jiangsu 212100 (CN)
(74) Representative: Hanna Moore + Curley
(86) International application number: PCT/CN2019/076799
(87) International publication number: WO 2020/073592

(57) **Abstract**

A pullulan empty hard capsule and a preparation method therefor. The empty hard capsule comprises the following components: 82-89 wt% of pullulan having a pH of 6-7, 1-1.4 wt% of gelling agent, 0.8-1.4 wt% of coagulant aid, 0.01-0.04 wt% of humectant, 0.01-0.04 wt% of surfactant, and 10-14wt% of purified water. The preparation method for the empty hard capsule comprises: weighing the components; dissolving the gelling agent, the coagulant aid, the humectant, the surfactant, a light-screening agent, a food coloring with 80-90°C purified water, putting the dissolved substances into a gel dissolving barrel, stirring till the gelling agent is dissolved, adding the pullulan having a pH of 6-7 while stirring, after the pullulan is completely dissolved, cooling, preparing a capsule by means of a glue dipping method, and drying.

## Description

### TECHNICAL FIELD

The present invention relates to the technical field of empty capsules, and in particular to a pullulan empty hard capsule and a preparation method therefor.

### BACKGROUND

Empty capsule is an oval empty shell made of specific main materials and auxiliary materials that can be used to hold a solid, a semi-solid, and a liquid. Traditional empty capsules use gelatin as the main material. Such empty capsules have better disintegration performance and fast drug release, but suffer from many problems. Gelatin is made by hydrolyzing materials such as the skin and bones of pig, cattle and other animals. It has a main component of collagen, and is easy to breed various microorganisms. It is a high-protein and high-fat substance, which is not conducive to the dietary health of patients, and may have safety issues caused by animal disease infection (such as mad cow disease, foot-and-mouth disease, avian flu, etc.). In addition, gelatin is derived from animals and is not suitable for vegetarians and ethnic and religious groups.

In order to solve the above-mentioned shortcomings, professionals have gone through a lot of research and developed new plant capsules to replace gelatin capsules. At present, capsules with the highest market share is the plant empty capsules with hypromellose and other cellulose and their derivatives as the main material, but such capsules have poor appearance transparency, delayed disintegration, and high oxygen permeability and other defects. Another new type of plant empty capsules uses pullulan as the main material. Compared with hypromellose empty capsules, such capsules have the advantages of good appearance transparency, fast disintegration speed, low oxygen permeability, etc.

Pullulan is an extracellular water-soluble mucinous polysaccharide obtained by fermentation of Aureobasidium pullulans. It cannot gel by itself, and requires the combined action of gelling agents such as carrageenan and gellan gum and coagulant aids such as cations, to form an edible film with low oxygen permeability and high solubility. It has been found during the development of pullulan empty capsules that the amount of gelling agents added has the greatest impact on the disintegration time of the capsules. The more the amount added, the longer the capsule disintegration time. The pH value of pullulan affects the amount of gelling agent added. The national standard stipulates that the pH value of pullulan is 4-8, and the pH value of pullulan suitable for preparing pullulan empty capsules is 6-7. Pullulan with a pH lower than that range becomes weaker in gelling strength in the process of combining with gelling agents and coagulant aids, so the amount of gelling agents added must be increased to ensure a fast gelling speed. However, the more the amount of gelling agents added is, the longer the disintegration time of the capsule is, and the more unfavorable the effect on drug release in the capsule is. The disintegration time of empty capsules made from pullulan raw materials having a pH of 6-7 is less than 10 min. Above that pH range, the viscosity of the glue solution will decrease rapidly with increased storage time of the glue solution, and the properties of the glue solution are unstable, which has a greater impact on dipping of capsules.

### SUMMARY

In view of the shortcomings in the prior art, the objective of the present invention is to provide a pullulan empty hard capsule and a preparation method therefor. The hard capsule has a short disintegration time, which is beneficial to the absorption of drugs.

A pullulan empty hard capsule comprises the following components: 82-89 wt% of pullulan having a pH of 6-7, 1-1.4 wt% of a gelling agent, 0.8-1.4 wt% of a coagulant aid, 0.01-0.04 wt% of a humectant, 0.01-0.04 wt% of a surfactant, and 10-14wt% of purified water.

As an improvement, the above-mentioned pullulan empty hard capsule further comprises a light-screening agent and a food coloring, wherein the content of the light-screening agent is less than 2.6 wt%, and the content of the food coloring is less than 1.7 wt%.

As an improvement, the gelling agent is carrageenan, the humectant is glycerin, the surfactant is sodium lauryl sulfate, the light-screening agent is titanium dioxide, and the coagulant aid is a cationic coagulant aid.

A preparation method for the above-mentioned pullulan empty hard capsule comprises the following steps:
step 1. weighing the pullulan, the gelling agent, the coagulant aid, the humectant, the surfacant, the light-screening agent, the food coloring, and the purified water;
step 2. dissolving the gelling agent, the coagulant aid, the humectant, the surfactant, the light-screening agent, and the food coloring with the purified water, transferring the dissolved substances into a gel dissolving barrel containing 80-90°C purified water, and stirring until the gelling agent is dissolved; adding the pullulan having a pH of 6-7 while stirring, and incubating at 80-90°C for 2-12 hour with stirring once every hour, and after the pullulan is completely dissolved, decreasing the temperature to 52-58°C; preparing a capsule by means of a glue dipping method and drying at 25-40°C, and obtaining a pullulan empty hard capsule when the moisture content of the hard capsule reaches 10-14% by drying.

As an improvement, in the step 2, the stirring speed is 1-5 rpm.

As an improvement, in the step 2, the moisture content of the pullulan empty hard capsule dried is 12.0-12.1%.

As an improvement, in the step 2, the temperature is decreased to 55°C.

### Advantageous Effects:

Compared with the prior art, the advantages of the present invention are that: 1. In view of the problems of long disintegration time and large fluctuation range of disintegration time among different batches in the existing pullulan capsule products, the hard capsules provided by the present invention significantly shorten the disintegration time by 6-8 minutes, and have stable disintegration time among different batches of capsules. 2. Pullulan used in the existing hard capsules has a pH value of 4-8. In order to meet the production needs, it is necessary to select the appropriate amount of gelling agents added according to the pH value of the pullulan, so that the component content of the hard capsules fluctuates greatly. In contrast, the product provided by the present invention limits the pH to a smaller range, so the added gelling agent is also in a smaller range, which effectively guarantees the component content of the hard capsule, and provides favorable conditions for administration to people of different physiques.

### DETAILED DESCRIPTION OF THE EMBODIMENTS

The present invention is further described in detail below by way of specific examples.

### Example 1

40 kg of 85°C purified water was added into a gel dissolving barrel. Another 8 kg of purified water was used to dissolve 192 g of carrageenan, 144 g of potassium chloride, 3.2 g of sodium lauryl sulfate, and 5.25 g of glycerin, and then they were poured into the gel dissolving barrel, and stirred until the carrageenan was dissolved, and 12 kg of pullulan having a pH value of 6.72 was slowly added while stirring. Then the glue solution was incubated at 85°C for about 4 h with stirring once every hour, and then cooled to 55°C for incubation and defoaming for later use.

In the production line, a capsule was prepared by means of a traditional dipping method and dried at 25-40°C, to obtain a transparent-appearance empty capsule with a moisture content of 12.1%, and the disintegration time was 7 min.

### Example 2

40 kg of 80°C purified water was added into a gel dissolving barrel. Another 8 kg of purified water was used to dissolve 192 g of carrageenan, 144 g of potassium chloride, 3 g of sodium lauryl sulfate, and 4.25g of glycerin, and then they were poured into the gel dissolving barrel, and stirred until the carrageenan was dissolved, and 12 kg of pullulan having a pH value of 6.63 was slowly added while stirring. Then the glue solution was incubated at 85°C for about 4 h with stirring once every hour, and then cooled to 55°C for incubation and defoaming for later use.

In the production line, a capsule was prepared by means of a traditional dipping method and dried at 25-40°C, to obtain a transparent-appearance empty capsule with a moisture content of 12.0%, and the disintegration time was 7 min.

### Example 3

40 kg of 88°C purified water was added into a gel dissolving barrel. Another 8 kg of purified water was used to dissolve auxiliary materials: 192 g of carrageenan, 144 g of potassium chloride, 2.3g of sodium lauryl sulfate, and 4.25 g of glycerin, and then they were poured into the gel dissolving barrel, and stirred until the carrageenan was dissolved, and 12 kg of pullulan having a pH value of 6.35 was slowly added while stirring. Then the glue solution was incubated at 85°C for about 4 h with stirring once every hour, and then cooled to 55°C for incubation and defoaming for later use.

In the production line, a capsule was prepared by means of a traditional dipping method and dried at 25-40°C, to obtain a transparent-appearance empty capsule with a moisture content of 12.0%, and the disintegration time was 7 min.

### Example 4

40 kg of 90°C purified water was added into a gel dissolving barrel. Another 8 kg of purified water was used to dissolve auxiliary materials: 192 g of carrageenan, 144 g of potassium chloride, 2.3 g of sodium lauryl sulfate, and 3.25 g of glycerin, and then they were poured into the gel dissolving barrel, and stirred until the carrageenan was dissolved, and 12 kg of pullulan having a pH value of 6.83 was slowly added while stirring. Then the glue solution was incubated at 85°C for about 4 h with stirring once every hour, and then cooled to 55°C for incubation and defoaming for later use.

In the production line, a capsule was prepared by means of a traditional dipping method and dried at 25-40°C, to obtain a transparent-appearance empty capsule with a moisture content of 12.1%, and the disintegration time was 7 min.

### Example 5

40 kg of 83°C purified water was added into a gel dissolving barrel. Another 8 kg of purified water was used to dissolve 192 g of carrageenan, 144 g of potassium chloride, 3.2 g of sodium lauryl sulfate, and 3.25 g of glycerin, and then they were poured into the gel dissolving barrel, and stirred until the carrageenan was dissolved, and 12 kg of pullulan having a pH value of 6.17 was slowly added while stirring. Then the glue solution was incubated at 85°C for about 4 h with stirring once every hour, and then cooled to 55°C for incubation and defoaming for later use.

In the production line, a capsule was prepared by means of a traditional dipping method and dried at 25-40°C, to obtain a transparent-appearance empty capsule with a moisture content of 12.1%, and the disintegration time was 7 min.

The above descriptions are merely preferred embodiments of the present invention, and are not intended to limit the protection scope of the present invention. Simple changes or equivalent replacements to the technical solutions readily apparent to any one skilled in the art within the technical scope disclosed in the present invention shall fall within the protection scope of the present invention.

## Claims

1. A pullulan empty hard capsule, comprising the following components: 82-89 wt% of pullulan having a pH of 6-7, 1-1.4 wt% of a gelling agent, 0.8-1.4 wt% of a coagulant aid, 0.01-0.04 wt% of a humectant, 0.01-0.04 wt% of a surfactant, and 10-14 wt% of purified water.

2. The pullulan empty hard capsule according to claim 1, further comprising an light-screening agent and a food coloring, wherein the content of the light-screening agent is less than 2.6 wt%, and the content of the food coloring is less than 1.7 wt%.

3. The pullulan empty hard capsule according to claim 1, wherein the gelling agent is carrageenan, the humectant is glycerin, the surfactant is sodium lauryl sulfate, the light-screening agent is titanium dioxide, and the coagulant aid is a cationic coagulant aid.

4. A method for preparing the pullulan empty hard capsule according to claim 1, comprising the following steps: step 1. weighing the pullulan, the gelling agent, the coagulant aid, the humectant, the surfacant, the light-screening agent, the food coloring, and the purified water; step 2. dissolving the gelling agent, the coagulant aid, the humectant, the surfactant, the light-screening agent, and the food coloring with the purified water, transferring the dissolved substances into a gel dissolving barrel containing 80-90°C purified water, and stirring until the gelling agent is dissolved; adding the pullulan having a pH of 6-7 while stirring, and incubating for 2-12 hour with stirring once every hour, and after the pullulan is completely dissolved, decreasing the temperature to 52-58°C; preparing a capsule by means of a glue dipping method and drying at 25-40°C, and obtaining a pullulan empty hard capsule when the moisture content of the hard capsule reaches 10-14% by drying.

5. The method for preparing a pullulan empty hard capsule according to claim 4, wherein in the step 2, the stirring speed is 1-5 rpm.

6. The method for preparing a pullulan empty hard capsule according to claim 4, wherein in the step 2, the moisture content of the pullulan empty hard capsule dried is 12.0-12.1%.

7. The method for preparing a pullulan empty hard capsule according to claim 4, wherein in the step 2, the temperature is decreased to 55°C.
